# EUROPEAN PATENT APPLICATION

(11) **EP 2 270 506 A2**
(43) Date of publication of application: **05.01.2011**
(21) Application number: 10166674.1
(22) Date of filing: 21.06.2010
(51) Int. Cl.: G01N 33/532, G01N 33/544, G01N 33/58

(54) **Amplified labeled conjugate for use in immunoassays**

(30) Priority: 02.07.2009 SE 0950519; 02.07.2009 US 222888 P
(71) Applicant: Amic AB, 751 83 Uppsala (SE)
(72) Inventor: Mendel-Hartvig, Ib, 756 55, Uppsala (SE); Rundström, Gerd, 752 41, Uppsala (SE)
(74) Representative: Carlsson, Carl Fredrik Munk

(57) **Abstract**

A conjugate, for use as a detection reagent in an immunoassay, is based on a hydrophilic monodisperse macromolecule, i.e. a macromolecule having a substantially uniform size and shape, said macromolecule forming a practically useful and manageable carrier, to which carrier at least one binding entity and at least one label are bound.

## Description

### Technical field

The present invention relates to the field of immunoassays, and in particular labeled conjugates consisting of at least one carrier and at least one binding entity for use in diagnostic assays.

### Background

Many assays comprise labeled reagents also referred to as detection conjugates, each label being provided to make it possible to qualitatively or quantitatively determine the presence of each particular reagent. Important labeling principles are based on color, luminescence, such as chemiluminescence and fluorescence, radioactivity, and magnetism. Fluorescent labels are frequently preferred, as such labels are easily available and amenable to fast, optical reading. Radioactive labels have the advantage of giving both a qualitative and a quantitative reading, but recent and current trends frequently favor non-radioactive labels.

There are mainly three important processes that drive the sensitivity of an assay with regards to the detection conjugate. These are the number of labels attached to the reagent, e.g. an antibody; the avidity of the detection conjugate, e.g. the number of antibodies per conjugate; and the concentration of the detection conjugate at the binding site, i.e. the number of possible binding events. One route to increase the sensitivity has been to use polymeric particles doped or labeled with high amounts of labels.

A known approach to increase labeling is to attach more labels to a particle, and attach the binding entity, e.g. an antibody, to such labeled particles.

Synthetic polymer particles, e.g. polystyrene particles, are available in different sizes. The size of fluorophore or chromophore doped polystyrene particles used today is most commonly larger than 100 nm in diameter.

Attaching labels to particles has been improved e.g. by coating the particles with suitable proteins exhibiting amino groups available for labeling. The following examples are given: thyroglobulin, bovine serum albumin, hemocyanin, myosin, apoferritin, catalase, a series of polylysine copolymers having different ratios of lysine/amino acid, [alpha]-2-macroglobulin, leucineaminopeptidase, heavy meromyosin and histones.

This strategy has in many cases been successful but has also given rise to problems with imprecision. Another problem with the latter approach is the low number of binding events at the binding sites due to limited number of particles that can be added in an assay. In practice, one particle label is considered to equal one binding entity, e.g. one antibody, in binding to the solid phase captured antigen. Furthermore, strong shear forces in flow assays might also affect the binding and stability of the sandwich formed.

US 5,891,741 concerns dextran crosslinked, antibody-phycobiliprotein conjugates containing up to twenty phycobiliprotein per dextran molecules. The disclosure mentions the term "monodisperse" but only in relation to one member of the phycobiliprotein family; phycoerythrin. The carrier used in US 5,891,741 is however dextran, which as such is polydisperse.

CA 1330061 discloses a detection conjugate comprising avidin or streptavidin linked to a carrier particle having more than fifteen amino groups on its surface, individually capable of being labeled with an operable label. The carrier particle is capable of being linked to avidin or streptavidin to form the detection conjugate.

US20050026305A1 concerns polydisperse aminodextran polymer molecules having a narrow size distribution, and their use as cell labeling reagents for flow cytometry.

For practical reasons, when using labeled particles according to the prior art, as exemplified in CA 1330061 and US20050026305A1, their concentration in solution must be limited.

One problem in the prior art is thus how to provide a conjugate which is manageable in higher concentrations.

Qin et al. (Anal Chem. 2001 Apr 1;73(7):1521-9) disclose two kinds of highly fluorescent streptavidin-based detection conjugates for use as universal detection reagents in ultrasensitive immunoassays. The direct conjugate was produced by covalently linking streptavidin to poly(Glu: Lys) which was labeled heavily with Eu chelates; the indirect conjugate was made by first conjugating bovine serum albumin (BSA) to poly(Glu:Lys) labeled heavily with Eu chelates and then further linking streptavidin to the conjugate of BSA-poly(Glu:Lys)-Eu chelate. Both direct and indirect conjugates were used to construct a highly sensitive time-resolved fluorometric assay for prostate-specific antigen (PSA).The assay using the direct conjugate was 1.5-fold more sensitive than a corresponding one using the indirect conjugate. When 45 microL of sample volume was used, a detection limit of 0.001 microg/L was achieved by using the direct conjugate. The conjugate according to Qin et al. is however not suitable for practical use, as the exact size of the polymer is difficult to control. In practice, the poly(Glu: Lys) polymer will be present in a large molecular weight span.

Another problem in the state of the art is the effects of using polydisperse polymers. A further problem is the low numbers of binding entities per detection conjugate and low binding avidity.

US 20060008206 relates to a waveguide plate and a process for its production and a microtiter plate comprising such a waveguide plate.

WO 2008/0733222 discloses indirect lateral flow sandwich assays, in which the target analyte binds to an analyte-specific reagent comprising a first member of a conjugate pair, forming a complex which contacts and binds a colored particulate label comprising a complimentary member of said conjugate pair, forming a second complex. Capture of this analyte-comprising, second complex by an immobilized analyte specific capture reagent results in the formation of an immobilized labeled sandwich complex that can be detected.

### Summary

It is an object of the present invention to obviate at least some of the disadvantages in the prior art and provide an improved conjugate.

One aim of the present invention is to make available amplified detection conjugates having an improved degree of labeling (DOL) without the disadvantages of using particles or polydisperse polymers as used in the background art.

Another aim is to make available conjugates with an increased number of binding entities per detection conjugate and thus increase the binding avidity of the complex.

Another aim is to utilize the improved detection conjugate for lateral flow assays and take advantage of the increased number of labels attached to the binding entity, the increased avidity and the possibility to make a high concentration of the detection conjugate available at the binding site.

Further aims behind the different embodiments of the invention, as well as advantages thereof will be evident upon a closer study of the background and the description of the invention.

The invention concerns a directly labeled detection conjugate where a binding entity is coupled to a labeled carrier molecule as defined in the independent claims. According to a preferred embodiment, both the carrier and the binding entity are labeled.

Methods for producing a labeled conjugate, as well as methods for performing an assay are defined in the independent claims, incorporated herein by reference.

In experiments involving embodiments of the invention, it has been shown that the degree of labeling (DOL) is significantly increased, the signal is improved and the sensitivity is increased when several binding entities, e.g. antibodies, are used in such detection conjugates.

The carrier molecule can include hydrophilic macromolecules. Preferred macromolecules include beta galactosidase, urease, keyhole limpet hemocyanine, and a streptavidin complex. Alternative carriers include natural and synthetic polyamino acids, peptides, proteins, nucleic acids, and carbohydrates, such as but not limited to hyaluronic acid. In one embodiment the hydrophilic monodisperse macromolecule is selected from the group consisting of a natural monodisperse hydrophilic polyamino acid, a synthetic monodisperse hydrophilic polyamino acid, a monodisperse hydrophilic peptide, a monodisperse hydrophilic protein, a monodisperse hydrophilic nucleic acid and a monodisperse hydrophilic carbohydrate.

One advantage of the present invention is that there is provided a conjugate which is manageable in higher concentrations, simplifying handling, deposition and drying.

Another advantage is that effects of polydisperse polymers are eliminated.

A further advantage is the possibility to significantly increase the number of binding entities per detection conjugate which increases the binding avidity of the complex.

A synergistic advantage is achieved when the improved detection conjugate is used in a lateral flow assay, taking advantage of the increased number of labels attached to the antibody, the increased avidity and the possibility to make a high concentration of the detection conjugate available at the binding site.

A further advantage is that the binding chemistry can be adjusted to the carrier, and the production simplified. One or more labels can be attached to the carrier molecule in a bulk reaction, producing a stock of labeled carrier. This labeled carrier stock is stable and can be stored. The binding entities can then be attached to portions of the labeled carriers, in reactions individualized to each binding element.

### Brief description of the drawings

The invention is now described, by way of example, with reference to the accompanying drawings, in which:

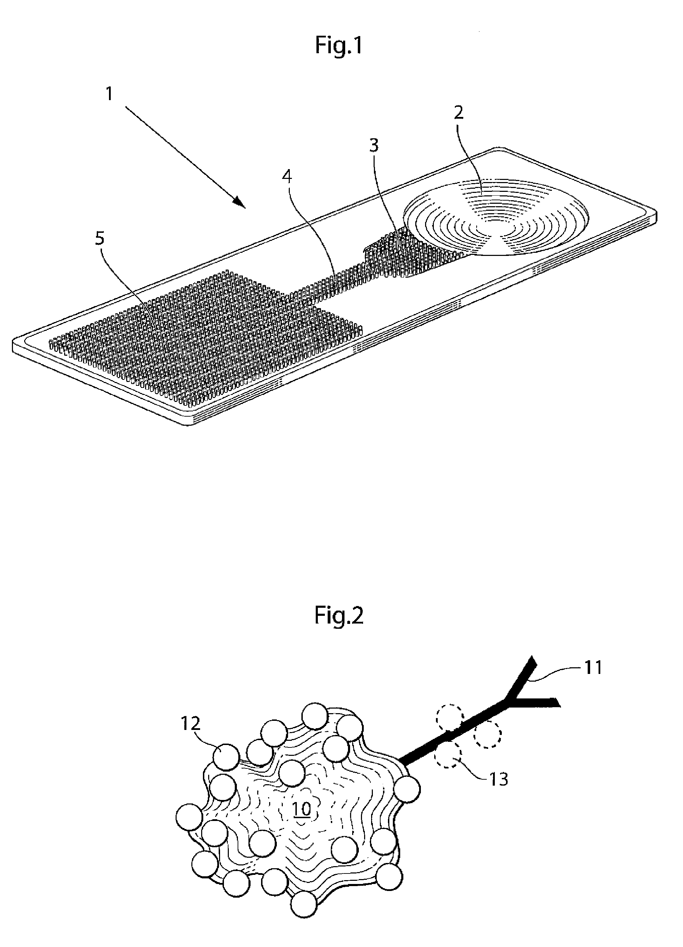

Fig. 1 schematically shows a lateral flow assay device, or so called assay chip 1, having a sample zone 2, a conjugate zone 3, a reaction zone 4, and a wicking zone 5, together forming a flow path extending from the sample zone 2 to the wicking zone 5. The reaction zone 4 may optionally comprise several reaction zones (not shown), located either subsequently in the direction of the flow, parallel or in the form of spots, in the flow path between the sample zone 2 and the wicking zone 5;
Fig. 2 schematically shows a labeled conjugate according to an embodiment of the invention, comprising a macromolecule 10, and bound thereto an binding entity, here schematically illustrated as an antibody 11. The macromolecule carries multiple labels 12, and, optionally, also the antibody 11 has labels 13 attached, here drawn with dashed lines; and

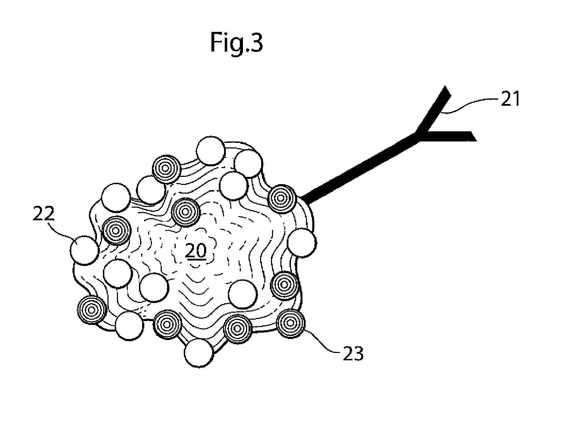

Fig. 3 schematically shows a labeled conjugate according to another embodiment of the invention, comprising a macromolecule 20, and bound thereto a binding entity, here shown as an antibody 21. The macromolecule carries two distinguishable labels 22 and 23.

### Detailed description

Before the invention is disclosed and described in detail, it is to be understood that this invention is not limited to particular compounds, configurations, method steps, substrates, and materials disclosed herein as such compounds, configurations, method steps, substrates, and materials may vary somewhat. It is also to be understood that the terminology employed herein is used for the purpose of describing particular embodiments only and is not intended to be limiting since the scope of the present invention is limited only by the appended claims and equivalents thereof.

It must be noted that, as used in this specification and the appended claims, the singular forms "a", "an" and "the" include plural referents unless the context clearly dictates otherwise.

If nothing else is defined, any terms and scientific terminology used herein are intended to have the meanings commonly understood by those of skill in the art to which this invention pertains.

The term "about" as used in connection with a numerical value throughout the description and the claims denotes an interval of accuracy, familiar and acceptable to a person skilled in the art. Said interval is ± 10 %.

The term "hydrophilic", refers to the capacity of a molecular entity to interact with polar solvents, in particular with water, or with other polar groups. Hydrophilic substances readily dissolve in water.

The term "monodisperse" or "mono-disperse" is used to define collection of objects, here termed "carriers" having uniform size and shape when discussing particles, and uniform mass, when discussing polymers. In the context of the present invention, uniform size, shape and/or mass indicated that the size, shape and/or mass is/are - for all practical purposes - identical within the group. A sample of objects, e.g. carriers that have an inconsistent size, shape and mass distribution are called polydisperse. A fraction, taken from a gradient, will always contain the gradient within that fraction.

Monodisperse collections can be created through the use of template-based synthesis, a common method of synthesis in nanotechnology. Monodisperse polymers are typically created through natural processes.

The term monodisperse is also used for polymer chains made by anionic polymerization, a method which results in chains of the same length. The catalyst used to initiate chains is anionic in nature. This technique is also known as living polymerization. It is used commercially for the production of block copolymers.

The term "macromolecule" is generally intended to mean a large molecule, and it is usually applied to the four conventional biopolymers; nucleic acids, proteins, carbohydrates, and lipids, as well as to non-polymeric molecules with large molecular mass. Macromolecules are synthesized through the process of polymerization, during which monomers are assembled into large molecules - macromolecules.

The term "binding entity" is used to define any molecule or moiety capable of specifically binding a component of interest for the purpose of detection the presence and optionally the concentration of said component in a sample. Examples of binding entities include, but are not limited to, the following: antibodies, antibody fragments, antibody haptens, aptamers, and nucleic acids, including DNA, PNA and LNA, protein receptors, ligand receptors, lectins and sugars.

The term "carrier" implies a particle or object which carries or forms the substrate or platform for other components, such as binding entities and labels, but which does not significantly participate in the reactions.

The term "conjugate" indicates the presence of several components, joined together, such as a carrier and a label, and optionally a carrier, label and binding entity.

The term "label" denotes a substance, molecule, particle or property, enabling the detection and/or identification a conjugate carrying said label.

One embodiment of the present invention is a conjugate for use as a detection reagent in an binding assay, said conjugate comprising at least one binding entity, at least one label and a carrier to which said at least one binding entity and at least one label are bound, wherein said carrier is a monodisperse macromolecule, e.g. a macromolecule having a substantially uniform size and shape, and that a plurality of said at least one label is bound to said macromolecule.

In the above embodiment, said at least one label is additionally bound also to said binding entity. Said macromolecule is preferably a macromolecule selected from the group consisting of natural or synthetic amino acids, including poly-amino acids, nucleic acids and carbohydrates.

Said macromolecule preferably is a hydrophilic monodisperse macromolecule, more preferably a hydrophilic monodisperse macromolecule having a molecular weight in the interval from about 50 kDa to about 10 000 kDa, more preferably in the interval from about 100 kDa to about 1 000 kDa.

In the above embodiment, said label is preferably selected from the group consisting of a luminescent compound, a radioactive compound, and an enzyme. Said binding entity is preferably selected from the group consisting of natural and synthetic antibodies, antibody fragments, antibody haptens, aptamers, and nucleic acids, including DNA, PNA and LNA, protein receptors, ligand receptors, lectins and sugars.

Another embodiment is a conjugate for use as a detection reagent in a binding assay, said conjugate comprising at least one binding entity, at least one first label and a second label and a carrier, wherein said carrier is a macromolecule, and a plurality of said first and second labels are bound to said macromolecule.

Preferably, said macromolecule is selected from the group consisting of a natural monodisperse hydrophilic polyamino acid, a synthetic monodisperse hydrophilic polyamino acid, a monodisperse hydrophilic peptide, a monodisperse hydrophilic protein, a monodisperse hydrophilic nucleic acid and a monodisperse hydrophilic carbohydrate.

In the above conjugate, said hydrophilic monodisperse macromolecule preferably has a molecular weight in the interval from about 50 kDa to about 10 000 kDa, more preferably in the interval from about 100 kDa to about 1 000 kDa.

According to a further embodiment of the invention, said first and second labels are distinguishable labels selected from the group consisting of a luminescent compound, a radioactive compound, a magnetic compound, and an enzyme. Preferably said first label is a fluorescent label and said second label is a fluorescent label different from said first label.

According to a specific embodiment, said first and second label are capable of charge transfer between the two optical active centers. Charge-transfer fluorescence probes show a strong response of the intensity and wavelength positions of their spectra to solvent polarity and mobility. Such properties have been applied in the past to follow polymerization kinetics and solvent-induced effects (e.g., swelling) in polymeric materials. An important advantage of charge-transfer fluorescence probes is that they can be measured at extremely low concentrations, hence interference with the polymeric system is limited. The construction of charge-transfer fluorescence probes is known to persons skilled in the art. A non-limiting example can be found in US 6818453, which discloses a label which is suitable for making a charge-transfer fluorescent probe, to a charge-transfer fluorescent probe comprising said label, to a diagnostic kit comprising the same, and to a method for detecting a material by using said charge-transfer fluorescent probe.

In the above conjugate, said binding entity is preferably selected from the group consisting of natural and synthetic antibodies, antibody fragments, haptens, aptamers, and nucleic acids, including DNA, PNA and LNA, protein receptors, ligand receptors, lectins and sugars.

Another embodiment of the invention is a method for labeling a binding entity, comprising the steps of:
- providing a carrier molecule, preferably a hydrophilic, monodisperse macromolecule;
- attaching a plurality of labels to said carrier, forming multiple labeled carrier molecules; and
- binding at least one binding entity to each carrier.

In the above method, the labeling of the carrier molecule is preferably performed under first conditions optimal for the binding of said label to said carrier, whereas the binding of said at least one binding entity is performed under second conditions, distinguishable from said first conditions, and optimal for the binding of said at least one binding entity to the labeled carrier molecule. The expression "optimal conditions for the binding" refers to physical and chemical conditions such as, but not limited to, temperature, pH, reagent concentration, buffer concentration, reaction time etc.

An example where different conditions are optimal is the case in which, for example, the label is bound to amine groups on the monodisperse macromolecule, the same coupling conditions for all conjugations, and the binding entity is bound to thiol groups on said macromolecule. The conditions for the thiol binding step can be fine-tuned depending on the identity of the binding entity. Another example is where the three-dimensional structure of the monodisperse macromolecule is influenced by the pH, wherein pH adjustments can be used to make different groups available for binding.

Further, the labeled carrier molecule can be stored after the step of attaching a plurality of labels to said carrier.

Another embodiment is a method for performing an immunoassay in an open lateral flow assay on a non-porous substrate, using a conjugate comprising at least one binding entity, at least one label and a carrier to which said at least one binding entity and at least one label are bound, wherein said carrier is a monodisperse macromolecule, e.g. a hydrophilic monodisperse macromolecule having a substantially uniform size and shape, and a plurality of said at least one label is bound to said macromolecule.

The above method for performing an immunoassay is further characterized by said conjugate being deposited in the conjugate zone (3) of a lateral flow assay device (1).

The use of monodispersed macromolecules, e.g. macromolecules having a substantially uniform and preferably also a well defined size and shape, is particularly useful in flow sandwich immunoassays, where the physical behavior of a particle can be a limiting factor on reaction kinetics, speed, sensitivity and accuracy of the assay.

The hydrophilic monodisperse macromolecule preferably has a molecular weight in the interval from about 50 kDa to about 10 000 kDa, more preferably in the interval from about 100 kDa to about 1 000 kDa.

Conventional particles having a diameter above 100 nm frequently exhibit impaired performance. Simultaneously, smaller particles have a strong tendency to aggregate and are thus no longer monodispersed in the reaction mixture. The use of polydisperse polymers with dissimilarity in size and net charge introduces variations in the assays and makes both manufacturing and control difficult. Surprisingly, initial tests using a system according to the invention has resulted in an amplified signal and increased sensitivity.

Effects of shear stress and low molar concentrations of the detection conjugate and other phenomena related to particles in flow assays are now at least partially alleviated or even completely eliminated. While substantially spherical, uniform and well defined with regard to size and shape, a macromolecule is also flexible, and thus adapts to the conditions in the lateral flow assay.

The use of a separate labeled carrier, to which at least one antibody is bound, makes it possible to separately optimize the labeling chemistry. The labeled carrier is then coupled to at least one antibody to be used in a detection conjugate with a variety of specificities.

In one embodiment of the invention two or more different carriers are used simultaneously in an assay, taking advantage of the inbuilt strengths of said carrier.

According to one embodiment of the invention, the labeled conjugate is applied to a lateral flow assay, for example an assay performed on a device as schematically shown in Fig. 1. This lateral flow assay device, or so called assay chip 1, has at least one sample zone 2, at least one conjugate zone 3, at least one reaction zone 4 optionally comprising several reaction zones, placed either subsequently in the direction of the flow, parallel or in the form of spots, in a flow path between the sample zone and a wicking zone 5. The flow path can comprise open or closed channels, grooves, capillaries. The flow path comprises a lateral flow path of adjacent projections, having a size, shape and mutual spacing such that capillary flow is sustained through said flow path. In one embodiment the flow path is at least partially open. In one embodiment the flow path is entirely open.

As used throughout the claims and the description the term "open" used in connection with capillary flow means that the system is open, i.e. the system is without a lid or cover entirely, or if there is a lid or a partial lid, said lid is not in capillary contact with the sample liquid, i.e. a lid shall not take part in creating the capillary force. An open lateral flow path is detailed in earlier applications by the same applicant, for example in the following published applications, incorporated herein by reference: WO 2003/103835, WO 2005/089082; WO 2005/118139; WO 2006/137785; and WO 2007/149042.

The labeled conjugate and methods according to embodiments of the invention can also be applied to other assay formats and assay devices, such as assays conducted on bibulous materials such as nitrocellulose fiber based materials. The labeled conjugate and methods according to embodiments of the invention can also be applied to assays conducted in suspension, in reaction vessels, or at least partially bound to the walls of reaction vessels, such as the wells of microtiter plates.

In contrast to known approaches involving the addition of an increasing number of fluorophores to a conjugate, the present invention is based on the joint effects of three elements: the binding entity, the label and the carrier. Using a truly monodisperse carrier molecule, any variability accountable to the carrier is minimized. Simultaneously, a monodisperse carrier according to the invention makes it possible to include both more labels and more binding entities in the conjugate. In a traditional conjugate, one binding entity is typically associated with 5 to 10 labels. In the conjugate according to the invention, multiple binding entities can be attached to the carrier, together with multiple labels. This results in a conjugate having a high degree of labeling, and thus improved signal, together with high avidity. In the background art, improving the signal is often a compromise between the degree of labeling, signal quenching, and avidity. When applying the present invention, using a separate labeled carrier, no negative effect on avidity is found.

An example of a conjugate made possible by the invention is monodisperse protein carrier, having at least 3 antibodies (binding entities), and at least 10 fluorophores (labels) per conjugate. As the carrier molecule is monodisperse, also the conjugate will for all practical purposes appear monodisperse, notwithstanding minor variations in the number of binding entities and labels. Preferably the number of binding entities, for example antibodies but not limited thereto, is from about 3 to about 10 or higher, per conjugate. Preferably the number of labels, for example fluorophores, but not limited thereto, is from about 10 to about 100, per conjugate.

A conjugate according to the invention is particularly advantageous in flow assays, where the time for binding between the binding entity and the analyte is short. Further, in a flow assay, a uniform particle size is significant for reliability and repeatability.

Other features and uses of the invention and their associated advantages will be evident to a person skilled in the art upon reading the description and the examples.

It is to be understood that this invention is not limited to the particular embodiments shown here. The following examples are provided for illustrative purposes and are not intended to limit the scope of the invention since the scope of the present invention is limited only by the appended claims and equivalents thereof.

### Examples

### Example 1

The test was carried out on a non-porous carrier, having a micropillar array defining an open lateral flow path, as described for example in one of WO 2003/103835, WO 2005/089082; WO 2005/118139; WO 2006/137785; and WO 2007/149042.

Plastic substrate chips made of Zeonor® (Zeon Corporation, Japan) were manufactured by Åmic AB, Uppsala, Sweden. The chips or assay devices were designed substantially as illustrated in Fig. 1.

The chips were given a coating of oxidized dextran on the surface for covalent immobilization of proteins via Schiff's base coupling. A reaction zone in the flow channel was deposited (AD3200 Biodot, USA) with 60 nl of 1 mg/ml anti-NT-proBNP (cl.13G12 Hytest Ltd, Finland) in 50 mM phosphate buffer pH 7.5, 1 % Trehalos.

The chips were dried 1 hr at 20% humidity and 30°C. Three different detection conjugates were used for comparison of the amplification strength of the beta-galactosidase-antibody complex. The complex was produced by means of thiol chemistry. The anti-NT-proBNP mAb cl. 1 5C4 (Hytest Ltd, Finland) was reacted with the bi-functional coupling reagent SPDP (N -Succinimidyl 3-(2-pyridyldithio)-propionate), (Pierce, Thermo Fisher Scientific, USA) to introduce a reactive disulfide. The activated antibody was conjugated to some of the native thiol groups of the enzyme beta-D-galactosidase, kindly provided by Phadia AB, Sweden. The rest of the thiol groups on the enzyme were conjugated to the activated dye Alexa Fluor 647 C2 Maleimide (Molecular Probes, Invitrogen, USA). The complex was finally purified on a Superose® column (GE Healthcare Life Sciences, Uppsala, Sweden) in 50 mM sodiumphosphate pH 7.5, 2 mM MgC12, 0.15 M NaCl, 0.02 % NaN3.

Two batches of control conjugates were produced (cl. 15C4). The detection conjugates were fluorescently labeled according to the supplier's instructions using Alexa Fluor® 647 Protein Labeling Kit (Invitrogen, USA). The three detection conjugates (1.35 µl of 0.25 mg/ml in 50 mM Borate buffer pH 8.5, 20% Trehalos, 2% BSA and 0.01 % 15 Tween 20) were deposited (Biodot 3200) in the conjugate zone (see fig. 1, feature 3).The chips were dried 1 hr in 20% relative humidity and 30°C.

The three detection conjugates were tested and run in the chip by adding 33 µl of plasma sample at two different concentrations of NT-proBNP to the sample zone of the chip. The capillary action of the micro post array distributed the sample across the flow channel dissolving the dried detection conjugate and transporting the sample conjugate mixture to the reaction zone and finally into the wicking zone. Surplus reagents in the flow channel were washed by the excess of sample without any conjugate. A typical assay time was about 10 minutes. The signal intensities were recorded in a prototype line-illuminating fluorescence scanner (Åmic AB, Uppsala, Sweden). A new chip was used for each assay and 2-replicates were used at each concentration. The results from the experiment are shown in Table 1.

**Table 1. Amplification by improved detection conjugate**

| Relative signal | | Amplification factor | |
|---|---|---|---|
| 300ng/ml | 3000ng/ml | 300 ng/ml | 3000 ng/ml |
| 690 | 4030 | NA | NA |
| 190 | 1090 | 3.6 | 3.7 |
| 130 | 900 | 5.3 | 4.8 |
| 160 | 995 | 4.3 | 4.1 |

Table 1 shows a comparison between a fluorescently labeled complex of antibodies and beta-galactosidase according to an embodiment of the invention, and directly labeled antibodies (controls 1 and 2).

As seen in Table 1, an amplification of the fluorescence signal by a factor of approximately 4 to 5 was found with the beta-Gal/Ab detection conjugate compared to the directly labeled antibody. The increase in sensitivity was even higher if the lower background binding when using the beta-Gal/Ab conjugate was used in the calculation.

### Example 2

The method of Example 1 is repeated using the same plastic substrate chips made of Zeonor® (Zeon Corporation, Japan) and the same coating and handling steps. Instead of beta-D-galactosidase, monodisperse urease was used as the carrier macromolecule. Urease, similarly to beta-D-galactosidase, has thiol groups available for conjugation of a binding entity, e.g. an antibody.

### Example 3

The method of Example 1 is repeated, using the same plastic substrate chips made of Zeonor® (Zeon Corporation, Japan) and the same coating and handling steps. As in Example 1, beta-D-galactosidase is used as the hydrophilic monodisperse carrier.

Unlike Example 1, two independent labels are used, for example two fluorophores which can be detected independently from each other, without the signals interfering. Another example of independent labels are labels that are detected using different physical principles. For example, a fluorophore and a radioactive label can be used, or a fluorophore and a magnetic label, or a magnetic label and a radioactive label.

The first label is conjugated to the monodisperse carrier, while the second label is conjugated to the binding entity. A conjugate is then formed by combining the monodisperse carrier and binding entity, each carrying their labels, under conditions suitable for forming the detection conjugate. The formation of a detection conjugate can then be verified, and the proportion of binding entity to carrier can be determined by determining the quotient of the signal from the first label and the signal from the second label.

The use of two independent labels can thus be applied to quality control and confirmation of successful formation of the detection conjugate.

### Example 4

The consecutive labeling of the monodisperse carrier macromolecule and the conjugation of a binding entity to form the detection conjugate can be investigated by performing the labeling of the carrier macromolecule under first conditions (pH, temperature, time) optimal for the formation of covalent bonding between the label and amine groups on the carrier molecule, and then forming the detection conjugate under second conditions (pH, temperature, time etc), different from said first conditions, and optimal for the covalent bonding between the binding entity and thiol groups on the carrier molecule.

The influence of pH on the three-dimensional structure of the carrier and as a consequence thereof, on the degree of labeling, can be investigated for a monodisperse macromolecule by varying the pH, adding the label, washing and determining the signal.

## Claims

1. A conjugate for use as a detection reagent in an binding assay, said conjugate comprising at least one binding entity, at least one label and a carrier to which said at least one binding entity and at least one label are bound, **characterized in that** said carrier is a hydrophilic monodisperse macromolecule having a molecular weight in the interval from about 50 kDa to about 10 000 kDa, and that a plurality of said at least one label is bound to said macromolecule.

2. The conjugate according to claim 1, wherein said binding entity also carries at least one label.

3. The conjugate according to claim 1, wherein said hydrophilic monodisperse macromolecule is selected from the group consisting of a natural monodisperse hydrophilic polyamino acid, a synthetic monodisperse hydrophilic polyamino acid, a monodisperse hydrophilic peptide, a monodisperse hydrophilic protein, a monodisperse hydrophilic nucleic acid and a monodisperse hydrophilic carbohydrate.

4. The conjugate according to claim 1, wherein said hydrophilic monodisperse macromolecule has a molecular weight in the interval from about 100 kDa to about 1000 kDa.

5. The conjugate according to claim 1, wherein said label is selected from the group consisting of a luminescent compound, a radioactive compound, a magnetic compound, and an enzyme.

6. The conjugate according to claim 1, wherein said binding entity is selected from the group consisting of an antibody, an antibody fragment, an antibody, a hapten, an aptamer, a nucleic acid, DNA, PNA, LNA, a protein receptor, a ligand receptor, a lectin and a sugar.

7. A conjugate for use as a detection reagent in an immunoassay, said conjugate comprising at least one binding entity, at least one first label and a second label and a carrier, **characterized in that** said carrier is a hydrophilic monodisperse macromolecule having a molecular weight in the interval from about 50 kDa to about 10 000 kDa, and that a plurality of said first and second labels are bound to said macromolecule.

8. The conjugate according to claim 7, wherein said hydrophilic monodisperse macromolecule is selected from the group consisting of a natural monodisperse hydrophilic polyamino acid, a synthetic monodisperse hydrophilic polyamino acid, a monodisperse hydrophilic peptide, a monodisperse hydrophilic protein, a monodisperse hydrophilic nucleic acid and a monodisperse hydrophilic carbohydrate..

9. The conjugate according to claim 7, wherein said hydrophilic monodisperse macromolecule has a molecular weight in the interval from about 100 kDa to about 1 000 kDa.

10. The conjugate according to claim 7, wherein said first and second labels are distinguishable labels selected from the group consisting of a luminescent compound, a radioactive compound, a magnetic compound, and an enzyme.

11. The conjugate according to claim 7, wherein said first label is a fluorescent label and said second label is a fluorescent label different from said first label.

12. The conjugate according to claim 11, wherein said first and second label are capable of charge transfer between the two optical active centers.

13. The conjugate according to claim 11, wherein said binding entity is selected from the group consisting of an antibody, an antibody fragment, an antibody hapten, an aptamer, a nucleic acid, DNA, PNA, LNA, a protein receptor, a ligand receptor, a lectin, and a sugar.

14. A method for labelling a binding entity, comprising the steps of:
- providing a carrier molecule selected from hydrophilic monodisperse macromolecules having a molecular weight in the interval from about 50 kDa to about 10 000 kDa;
- attaching a plurality of labels to said carrier, forming multiple labelled carrier molecules; and
- binding at least one binding entity to each carrier.

15. The method according to claim 14, wherein the labelling of the carrier molecule is performed under first conditions optimal for the binding of said label to said carrier, whereas the binding of said at least one binding entity is performed under second conditions, distinguishable from said first conditions, and optimal for the binding of said at least one binding entity to the labelled carrier molecule.

16. The method according to any one of claims 14 and 15, wherein said method comprises a step of storing the labelled carrier molecule after the step of attaching a plurality of labels to said carrier, and before binding at least one binding entity to each carrier.

17. A method for performing an binding assay in an open lateral flow assay on a non-porous substrate, using a conjugate comprising at least one binding entity, at least one label and a carrier to which said at least one binding entity and at least one label are bound, **characterized in that** said carrier is a hydrophilic monodisperse macromolecule having a molecular weight in the interval from about 50 kDa to about 10 000 kDa, and a plurality of said at least one label is bound to said macromolecule.

18. The method according to claim 17, wherein said conjugate is deposited in the conjugate zone (3) of a lateral flow assay device (1).
